# EUROPEAN PATENT APPLICATION

(11) **EP 4 606 407 A1**
(43) Date of publication of application: **27.08.2025**
(21) Application number: 23879078.6
(22) Date of filing: 16.10.2023
(51) Int. Cl.: A61M 15/00

(54) **MEDICATION DISPENSER**

(30) Priority: 20.10.2022 CN 202211287312
(71) Applicant: Transpire Bio Inc, Plantation, FL 33324 (US)
(72) Inventor: LIU, Yongwei, Shenzhen, Guangdong 518102 (CN); ZHANG, Xieen, Shenzhen, Guangdong 518102 (CN); OUYANG, Guanglin, Shenzhen, Guangdong 518102 (CN)
(74) Representative: Latscha Schöllhorn Partner AG
(86) International application number: PCT/CN2023/124817
(87) International publication number: WO 2024/083089

(57) **Abstract**

This disclosure relates to a medicine dispenser comprising a housing with an opening; and a cover assembly configured for the opening and movably mounted on the housing, the cover assembly including: a first cover pivotally mounted on the housing; and a second cover arranged inside the first cover, where the second cover is configured to be capable of being driven by the first cover to rotate.

## Description

### CROSS-REFERENCE TO RELATED APPLICATIONS

This application claims priority to Chinese Patent Application No. 202211287312.6 filed on October 20, 2022, which is incorporated herein by reference in its entirety.

### TECHNICAL FIELD

This application is generally related to medical inhalers, and, more specifically, to dry powder medical inhalers.

### BACKGROUND

Each known medicine dispenser for inhalation administration is provided with an opening for a user to inhale medicine. The opening is provided with a cover to prevent contamination. In use, the cover needs to be moved away from the opening to expose the opening. However, the cover is often unintentionally moved, causing the opening to be unexpectedly exposed, thus posing a risk of contamination to the opening.

### SUMMARY

The purpose of this disclosure is to provide a medicine dispenser that can at least partially solve the above problems. This disclosure provides a medicine dispenser, including: a housing provided with an opening; a dispensing mechanism configured to dispense medicine and arranged on the housing; and a cover assembly configured for the opening and movably mounted on the housing, the cover assembly including: a first cover pivotally mounted on the housing; and a second cover arranged inside the first cover, where the second cover is configured to be capable of being driven by the first cover to rotate.

According to an embodiment of this disclosure, at least before the second cover is driven by the first cover, the dispensing mechanism is not triggered.

According to an embodiment of this disclosure, the housing is provided with a stop for the first cover to provide a first stop position and a second stop position for the first cover, and at least in the first stop position, the first cover completely covers the second cover.

According to an embodiment of this disclosure, the second cover is pivotally mounted on the housing, and when the first cover is about to move to the second stop position, the rotational movement of the second cover is triggered by a trigger unit of the medicine dispenser.

According to an embodiment of this disclosure, the trigger unit includes a torsion spring, two torsion arms of the torsion spring are effectively connected to the first cover and the second cover respectively, and the torsion spring is configured to respectively apply forces in opposite directions along the circumferential direction to the first cover and the second cover.

According to an embodiment of this disclosure, the trigger unit includes a cantilever type trigger arm, the trigger arm is elastically deformable and provided with a first fit protrusion assigned to a first protrusion of the first cover and a second fit protrusion assigned to a second protrusion of the second cover, and the first fit protrusion and the second fit protrusion are spaced apart from each other.

According to an embodiment of this disclosure, in the first stop position, the second protrusion of the second cover is pressed against the second fit protrusion of the trigger arm; and
according to an embodiment of this disclosure, in the second stop position, the first protrusion of the first cover is pressed against the first fit protrusion of the trigger arm.

According to an embodiment of this disclosure, the trigger unit includes an actuator non-rotatably connected to the first cover and a driving ring non-rotatably connected to the second cover, the actuator is provided with a first notch for a first torsion arm of the torsion spring, and the driving ring is provided with a second notch for a second torsion arm of the torsion spring.

According to an embodiment of this disclosure, the side of the second fit protrusion facing toward the first fit protrusion is configured to be an inclined plane, and/or the side of the second protrusion of the second cover facing away from the second fit protrusion is configured to be an inclined plane.

According to an embodiment of this disclosure, the trigger arm is configured at a retaining plate mounted on the housing.

According to an embodiment of this disclosure, the first cover is configured to be U-shaped, and the second cover is configured to be hook-shaped. According to an embodiment of this disclosure, when the first cover moves to be partially misaligned with the second cover, the movement of the second cover is triggered by the trigger unit of the medicine dispenser.

According to an embodiment of this disclosure, the second cover is movably retained on the inner side of the first cover relative to the first cover.

According to an embodiment of this disclosure, the first cover and the second cover are respectively provided with an arc-shaped groove and a first bulge that are fit with each other, and the first bulge is arranged in the arc-shaped groove.

According to an embodiment of this disclosure, the cover with the first bulge is further provided with a second bulge, the second bulge is arranged to be capable of entering and exiting the groove, and referring to the opening direction of the medicine dispenser, the circumferential distance between the front end portion of the groove and the front edge of the cover with the arc-shaped groove is equivalent to the circumferential distance between the first bulge and the second bulge.

According to an embodiment of this disclosure, the first bulge is cylindrical, and the end surfaces of at least two consecutive end sides of the second bulge along the opening direction are inclined planes.

According to an embodiment of this disclosure, the housing is provided with a limiting structure for the second cover, and the second cover is provided with a fit limiting structure corresponding to the limiting structure.

By adopting the medicine dispenser provided in this disclosure, especially by arranging two covers for the medicine dispenser, the opening of the medicine dispenser can be effectively prevented from being unexpectedly exposed since the cover is accidentally opened, thus preventing the opening from being contaminated. At the same time, the provided medicine dispenser has a simple structure, is easy to mount, and is easy to operate.

The features and advantages of this disclosure will become clearer from the brief description of the drawings, the drawings, the detailed description of the exemplary embodiments, and the attached claims below.

### BRIEF DESCRIPTION OF THE DRAWINGS

This disclosure will be further described below with reference to the drawings. In the figures:
FIG. 1 is a perspective view of a medicine dispenser according to this disclosure, where the medicine dispenser is in a first state;
FIG. 2 is an exploded diagram of a first side of a medicine dispenser according to a first embodiment of this disclosure;
FIG. 3 is an exploded diagram of a second side of a medicine dispenser according to the first embodiment of this disclosure;
FIG. 4 is a partial enlarged diagram of the medicine dispenser in FIG. 2;
FIG. 5 is an exploded diagram of a part of a trigger unit of a medicine dispenser according to the first embodiment of this disclosure;
FIG. 6 is a partial enlarged diagram of a first cover of a medicine dispenser according to thr first embodiment of this disclosure;
FIG. 7 is a partial enlarged diagram of a second cover of a medicine dispenser according to the first embodiment of this disclosure;
FIG. 8 is a three-dimensional diagram of a part of the medicine dispenser in FIG. 1, where a housing is omitted;
FIG. 9a to FIG. 9c respectively are three-dimensional diagrams of a medicine dispenser in a second state according to the first embodiment of this disclosure;
FIG. 10a to FIG. 10c respectively are three-dimensional diagrams of a medicine dispenser in a third state according to the first embodiment of this disclosure;
FIG. 11a to FIG. 11c respectively are three-dimensional diagrams of a medicine dispenser in a fourth state according to the first embodiment of this disclosure;
FIG. 12 is a three-dimensional diagram of a medicine dispenser in an alternative fourth state according to the first embodiment of this disclosure;
FIG. 13 is an exploded diagram of a medicine dispenser according to a second embodiment of this disclosure;
FIG. 14 is another exploded diagram of a medicine dispenser according to the second embodiment of this disclosure;
FIG. 15a to FIG. 15d respectively illustrate diagrams of a medicine dispenser in different states according to the second embodiment of this disclosure;
FIG. 16 schematically is another three-dimensional diagram of a medicine dispenser according to this disclosure, where a dispensing mechanism arranged in a housing of the medicine dispenser is illustrated;
FIG. 17 schematically is an exploded diagram of a dispensing mechanism arranged in a housing of a medicine dispenser; and
FIG. 18a to FIG. 18c respectively illustrate diagrams of a dispensing mechanism in different positions.

### DETAILED DESCRIPTION

Exemplary embodiments of this disclosure will be described in more detail below with reference to the drawings. Although the exemplary embodiments of this disclosure are shown in the drawings, it is to be understood that this disclosure can be implemented in various forms and is not limited to the embodiments described herein. On the contrary, these embodiments are provided to make this disclosure more thorough and complete, and to fully convey the scope of this disclosure to those skilled in the art.

In the following description, some specific details are elaborated for the purpose of describing various embodiments of this disclosure to provide a thorough understanding of the various embodiments of this disclosure. However, those skilled in the art will recognize that the embodiments be practiced without one or more of these specific details. In other cases, well-known apparatuses, structures, and techniques associated with this application can not be shown or described in detail to avoid unnecessary confusion with the description of the embodiments.

Unless the context requires otherwise, throughout the entire description and claims, the words "include" and its variations, such as "comprising" and "having", should be understood as having an open and inclusive meaning, that is, should be interpreted as "including, but not limited to".

The mention of "an embodiment" or "some embodiments" throughout the entire description represents that specific characteristics, structures, or features described in conjunction with the embodiments are included in at least one embodiment. Therefore, the appearance of "in an embodiment" or "in some embodiments" in various positions throughout the entire description can not necessarily refer to the same embodiment. In addition, specific characteristics, structures, or features can be combined in any way in one or more embodiments.

In addition, terms such as "first" and "second" used in the description and claims are only intended to distinguish between objects for clarity of description, and do not limit the size, quantity, or other order of the objects described.

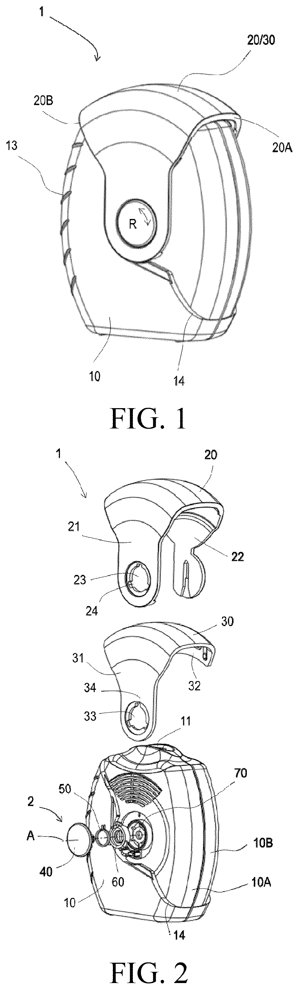

FIG. 1 is a three-dimensional diagram of a medicine dispenser 1 according to embodiments disclosed herein. The medicine dispenser 1 comprises a housing 10. The housing 10 is provided with an opening 11. An object to be positioned in or an object to be removed can be positioned in or removed from the housing 10 through the opening 11. Exemplary objects can be in a solid, liquid, fluid or other suitable state, such as water, a beverage, a powder, a pill, or an ointment. The medicine dispenser 1 further includes a cover assembly. The cover assembly is configured to be positioned proximate to opening 11, that is, the cover assembly can be configured to cover or seal the opening 11. The cover assembly is movably mounted on the housing 10. The cover assembly can comprise a first cover 20 and a second cover 30. The first cover 20 is pivotally mounted on the housing 10. The second cover 30 is positioned inside the first cover 20.

The first cover 20 is configured to drive the second cover 30 to move, such as through a pivoting motion. Therefore, each of the covers can have a relatively smaller circumferential dimension, thus enhancing the design freedom of the medicine dispenser 1, especially the housing 10 of the medicine dispenser 1. As described in more detail herein, through such arrangement, it is possible to reduce or avoid unintentional uncovering of opening 11 to at least some extent.

As illustrated in FIG. 1, the cover assembly can pivot in the direction indicated by the double arrow R to release or cover the opening 11. As can also be seen from FIG. 1, in this state of the medicine dispenser, i.e., in a first state, the first cover 20 completely covers the second cover 30. In this case, it can be considered that the second cover 30 has retracted into a space between the first cover 20 and the housing 10 such that the second cover 30 is nested under the first cover 20. Therefore, the outer contoured surface of the second cover 30 is not greater than the inner contoured surface of the first cover 20, which makes the overall structure of the medicine dispenser more compact and achieves a better visual effect.

At least one of the two side surfaces of the housing 10 perpendicular to a pivoting axis A of the cover assembly is provided with a step portion. The step portion forms a stop 14 for the first cover 20, so as to limit the terminal position of the first cover 20 in the circumferential direction through the stop 14, that is, to provide a first stop position and a second stop position for the first cover 20, to achieve the restricted movement of the cover assembly. Preferably, the contour direction of the stop 14 corresponds, at least in some sections, to the contour direction of the front edge 20A and the rear edge 20B of the reference direction R of the first cover 20. In a first state of the medicine dispenser illustrated in FIG. 1, that is, in a first stop position of the first cover 20, the first cover 20 completely covers the second cover 30.

Stop 14 of the housing 10 can be provided with an opening for at least a portion of a side wall of the first cover 20 to extend into the housing. For example, the side wall of the first cover 20 extending into the housing can cooperate with other members arranged in the housing to achieve the desired function.

In a first embodiment of the medicine dispenser 1 disclosed herein, referring to FIGS. 2-7, the first cover 20 is configured to be inverted-U-shaped, two side walls 21, 22 are spaced apart, and the housing 10 is partially located between the inwardly facing surfaces of the two side walls 21, 22. The second cover 30 is substantially configured to be hook-shaped, and has only one side wall 31 with a length substantially corresponding to the length of the side wall of the first cover 20 and another side wall 32 with the length which is significantly shorter than side wall 31. The side wall 31 is arranged between the side wall 21 of the first cover 20 and the side surface of the housing 10. The side wall 21 of the first cover 20 and the side wall 31 of the second cover 30 are configured to cooperate with a trigger unit of the medicine dispenser, which will be further described below.

The trigger unit 2 includes a torsion spring 50. The torsion spring 50 has two torsion arms 51, 52 and a cylindrical spiral body 53 located between them. A hole 55 is defined through the spiral body. The torsion spring 50 is configured to respectively apply forces in opposite directions along the circumferential direction to the first cover 20 and the second cover 30. Preferably, the above forces are proportional to the amount of torsion of the torsion spring 50. The torsion arms 51, 52 of the torsion spring 50 can be effectively connected to the first cover 20 and the second cover 30 respectively, for example, through force transmission connection, especially indirect force transmission connection.

In the illustrated embodiment, the torsion arms 51, 52 of the torsion spring 50 are effectively connected to the first cover 20 and the second cover 30 respectively, through corresponding intermediate members. Specifically, an actuator 40 is assigned to the first cover 20 and is non-rotatably connected to the first cover 20; and a driving ring 60 is assigned to the second cover 30 and is non-rotatably connected to the second cover 30.

An end portion of the side wall 21 of the first cover 20 is provided with a first interface 23. The inner periphery of the first interface is provided with first recesses 24. The actuator 40 is correspondingly provided with first bosses 43. In an assembled state, the first bosses 43 are positioned in the first recesses 24, thus establishing a shape fit connection between the first cover 20 and the actuator 40. Exemplarily, the first cover 20 is provided with three square first recesses distributed along the circumferential direction, and the actuator 40 is provided with three square first bosses distributed along the circumferential direction. One of the first bosses 43 of the actuator 40 is provided with a first notch 44. The first notch 44 is configured to receive the first torsion arm 51 of the torsion spring 50. This configuration is exemplary, and a skilled artisan would recognize other numbers and shapes of bosses or recesses are also contemplated.

The side wall 22 of the first cover 20 is provided with an actuating structure 27, for example in the form of an elongated hole. The actuating structure 27 is effectively connected to a dispensing mechanism 300 that will also be described in more detail below, so that the dispensing mechanism 300 can be triggered at the appropriate time via the first cover 20 to dispense medicine. In other embodiments, the actuating structure 27 can be any other form of non-circular hole or any other suitable structure readily contemplated by a skilled artisan.

An end portion of the side wall 31 of the second cover 30 adjacent to the side wall 21 of the first cover 20 is provided with a second interface 33. An inner periphery of the second interface 33 is provided with a second recess 34. The driving ring 60 is correspondingly provided with a second boss 63. In an assembled state, the second boss 63 is positioned in the second recess 34, thus establishing a shape fit connection between the second cover 30 and the driving ring 60. Exemplarily, the second cover 30 is provided with three square second recesses distributed along the circumferential direction, and the driving ring 60 is provided with three square second bosses distributed along the circumferential direction. One of the second bosses 63 of the driving ring 60 is provided with a second notch 64. The second notch 64 is configured to receive the second torsion arm 52 of the torsion spring 50. In other embodiments, it is contemplated that 3, 4, or more bosses or recesses are also feasible, with other shapes also being possible.

When manipulating the first cover 20, the force applied to the first cover 20 can be transmitted to the first torsion arm 51 of the torsion spring 50 via the actuator 40, and then transmitted to the second cover 30 through the second torsion arm 52 of the torsion spring 50. When the trigger condition is met, the mechanical energy stored in the torsion spring 50 causes the movement of the second cover 30, thus opening the opening.

In order to retain the torsion spring 50 and the driving ring 60 at the housing 10 of the medicine dispenser, the actuator 40 can be approximately configured to be approximately thumb tack-shaped, that is, the actuator 40 can be provided with a disc portion 41 and a cylindrical portion 45 protruding from one side of the disc portion 41, the end portion of the cylindrical portion 45 is provided with at least one pair of fasteners 47, and the cylindrical portion 45 can be inserted into holes 55, 65 of the spiral body 53 of the torsion spring 50 and a ring body 61 of the driving ring 60, and locked at a bayonet 76 formed in the housing or in a member fixed on the housing through the fasteners. In addition, an annular portion 42 is positioned outside the radial direction of the cylindrical portion 45 in a manner of being spaced apart, and the first bosses 43 can be formed on the outer side of the radial direction of the annular portion 42. In some embodiments, multiple protrusions protruding inwards along the radial direction and distributed in the circumferential direction can be provided on the inner side of the radial direction of the annular portion 42, and the diameter of a circle defined by the end surfaces of the protrusions corresponds to the outer diameter of the spiral body 53 of the torsion spring 50. Therefore, the above protrusions can be configured at least partially to retain the torsion spring 50 during assembling to make the assembling easier.

The hole 65 of the driving ring 60 is a stepped hole, so that the end portion of the torsion spring 50 facing toward the driving ring 60 can be pressed against the step of the hole 65.

In some instances, the actuator 40 is integrally formed.

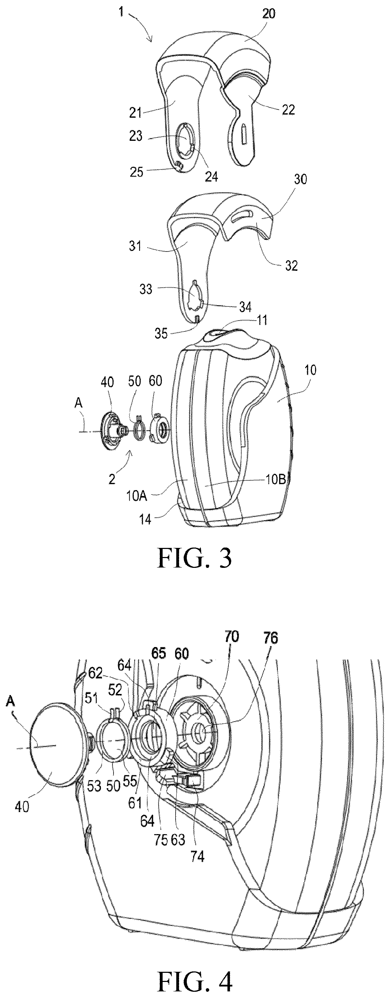

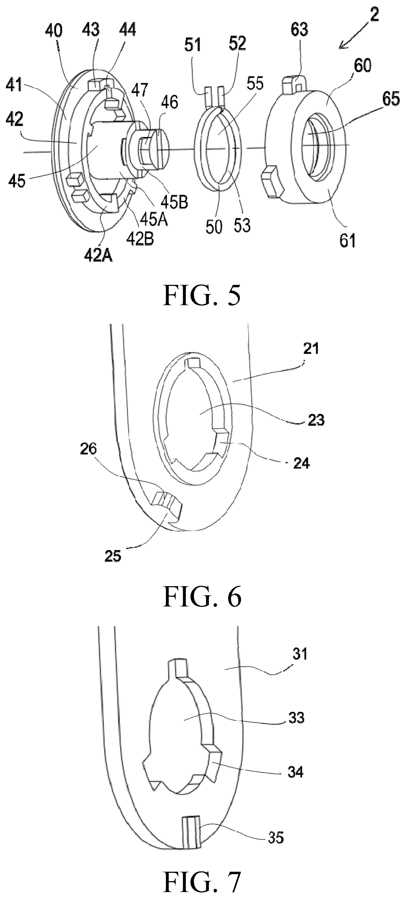

In some cases, the cylindrical portion 45 of the actuator 40 is configured in stages, that is, the cylindrical portion 45 can have a first cylindrical section 45A and a second cylindrical section 45B, and the first cylindrical section 45A and the second cylindrical section 45B have different diameters. In an illustrated example shown in at least FIGS. 4 and 5, the diameter of the first cylindrical section 45A is larger than the diameter of the second cylindrical section 45B. The inner diameter of the spiral body 53 of the torsion spring 50 is larger than the diameter of the first cylindrical section 45A, and the diameter of the hole section with the smallest inner diameter of the hole 65 of the driving ring 60 is equivalent to the diameter of the first cylindrical section 45A. Therefore, the torsion spring 50 can be retained between the actuator 40 and the driving ring 60.

In some embodiments, the annular portion 42 of the actuator 40 can also be configured in stages, that is, the annular portion 42 can have a first annular section 42A and a second annular section 42B, they have different axial heights, and thus a step is formed in the annular portion 42. Correspondingly, a lug 62 is provided on the side of the driving ring 60 facing toward the actuator 40. The step of the annular portion 42 of the actuator 40 and the lug 62 of the driving ring 60 are configured to be capable of being pressed against each other under the action of the torsion spring 50 in an initial state.

In some embodiments, the coils of the spiral body 53 of the torsion spring 50 can be spaced apart from each other. In the state where the actuator 40, the torsion spring 50, and the driving ring 60 are mounted on the housing, the torsion spring 50 is further subjected to the axial compression, thus applying the axial force to the driving ring 60, causing the driving ring 60 to press against the housing or related members fixed on the housing.

The bayonet 76 of the fastener 47 for the cylindrical portion 45 of the actuator 40 can be directly configured in the housing. Alternatively, a separate retaining plate 70 can also be provided, the retaining plate 70 is fixed to the housing, and the retaining plate 70 is provided with the bayonet 76 for the above fastener 47. To enhance the structural strength of the bayonet 76, a reinforcement rib can be provided for the bayonet or the thickness of the wall around the bayonet can be increased. By providing the separate retaining plate 70, the design and manufacturing of the housing 10 can be simplified.

In some instances, to facilitate the insertion of the fastener 47 of the cylindrical portion 45 of the actuator 40 into the bayonet more easily, the cylindrical portion 45 can be provided with a cutout 46 in the center, thus dividing at least a portion of the section of the cylindrical portion 45 into two parts, and the two parts can move towards each other under an external force and be reset after the external force is released.

The trigger unit further includes a cantilever type trigger arm 72, the trigger arm 72 is elastically deformable and provided with a first fit protrusion 75 assigned to a first protrusion 25 of the first cover 20 and a second fit protrusion 74 assigned to a second protrusion 35 of the second cover 30, and the first fit protrusion 75 and the second fit protrusion 74 are spaced apart from each other. In this case, the first fit protrusion 75 is arranged on a path that the first protrusion 25 rotates around the axis A, and the second fit protrusion 74 is arranged on a path that the second protrusion 35 rotates around the axis A.

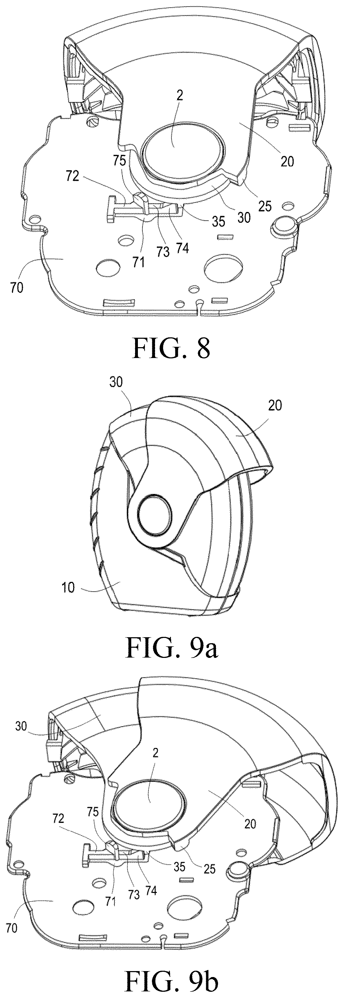

As can be seen from FIG. 8, the second protrusion 35 is located inside the radial direction of the first protrusion 25 relative to the axis A.

As can be seen from FIG. 3, FIG. 6, and FIG. 7, the first protrusion 25 and the second protrusion 35 protrude from corresponding side walls along a direction towards the internal space of the housing 10.

The trigger arm 72 can also be integral with retaining plate 70, thus reducing the number of components for medicine dispenser or simplifying the structure of the housing. For this purpose, a U-shaped notch 71 can be provided in the retaining plate 70 to form a cantilever beam structure. The first fit protrusion 75 and the second fit protrusion 74 are provided on the same side of an arm portion 73 of the trigger arm 72.

In some embodiments, the above bayonet 76 and the trigger arm 72 are integrally formed with the retaining plate 70, for example, through injection molding.

The working mode of the medicine dispenser according to a first embodiment of this disclosure will be described below with reference to the drawings. FIG. 1 to FIG. 5 and FIG. 8 illustrate the medicine dispenser in a first state, where the cover is in a first stop position. In the first stop position, under the action of the torsion spring 50, since forces in opposite directions along the circumferential direction are applied to the first cover 20 and the second cover 30, the first cover 20 is pressed against the stop 14, i.e., an upper part of the stop in FIG. 1, and the second protrusion 35 of the second cover 30 is pressed against the second fit protrusion 74 of the trigger arm 72. In order to apply the circumferential forces to the first cover 20 and the second cover 30, the torsion spring 50 is already under tension in the first state of the medicine dispenser. As can be seen from FIG. 5, for example, the circumferential spacing between the two torsion arms 51, 52 of the torsion spring 50 is almost zero. As can be seen from FIG. 8, in the opening direction, i.e., in the clockwise direction, the second protrusion 35 is arranged downstream of the first protrusion 25, and the first protrusion 25 and the first fit protrusion 75 are spaced apart from each other in the circumferential direction.

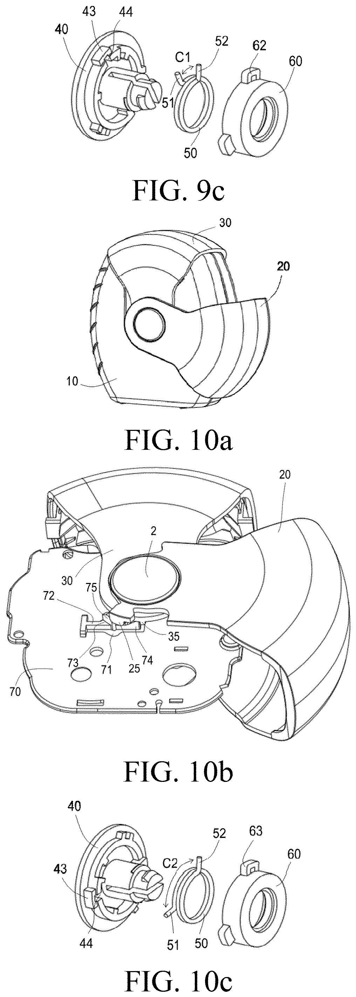

FIG. 9a to FIG. 9c illustrate the medicine dispenser 1 in a second state. It can be seen that the first cover 20 has rotated along the clockwise direction relative to the second cover 30. In the second state, the positions of the second cover 30 and the driving ring 60 are not changed relative to their positions in the first state. As can be seen from FIG. 9b, the first protrusion 25 of the first cover 20 is closer to the first fit protrusion 75 of the trigger arm 72 in the circumferential direction, and the second protrusion 35 of the second cover 30 is still pressed against the second fit protrusion 74 of the trigger arm 72.

The first torsion arm 51 of the torsion spring moves with the movement of the first cover 20 along the circumferential direction, while the second torsion arm 52 of the torsion spring remains stationary since the second cover 30 is stopped by the second fit protrusion 74. As a result, the circumferential distance between the torsion arms 51, 52 of the torsion spring increases to C1, that is, the torsion spring is further pre-tensioned, thus further increasing the energy stored in the torsion spring.

FIG. 10a to FIG. 10c illustrate the medicine dispenser 1 in a third state. At this time, the first cover 20 is about to move to the second stop position, that is, the pivotal movement of the second cover 30 is triggered by the trigger unit of the medicine dispenser through the first cover 20. Compared to the second state, in the third state, the first cover 20 further moves along the clockwise direction, causing the front edge of the first protrusion 25 of the first cover 20 to be in contact with the rear edge of the first fit protrusion 75 of the trigger arm 72. The second protrusion 35 of the second cover 30 continues to be pressed against the second fit protrusion 74 of the trigger arm 72. Therefore, the positions of the second cover 30 and the driving ring 60 are not changed. As can be seen from FIG. 10, the first torsion arm 51 of the torsion spring further moves with the movement of the first cover 20 along the circumferential direction, while the second torsion arm 52 of the torsion spring continuously remains stationary since the second cover 30 is stopped by the second fit protrusion 74. As a result, the circumferential distance between the torsion arms 51, 52 of the torsion spring increases to C2, where C2 is greater than C1. At this time, the energy stored in the torsion spring reaches its maximum. In this way, it avoids unintentionally revealing the opening, thus avoiding unexpected leakage of the object positioned in the medicine dispenser.

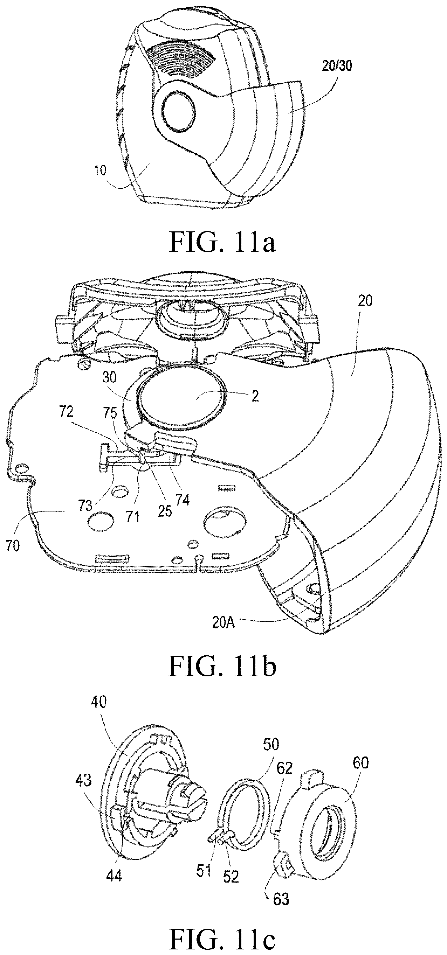

FIG. 11a to FIG. 11c illustrate the medicine dispenser 1 in a fourth state. The fourth state can also be referred to as the second stop position of the cover. At this time, the first cover 20 moves along the clockwise direction to the lower part of the stop 14 that is pressed against the housing 10, that is, the side surface 20A is pressed against the stop 14. During this period, the first protrusion 25 of the first cover 20 slides onto the top of the first fit protrusion 75 of the trigger arm 72, thus pressing the first fit protrusion 75 downward towards the internal space of the housing, causing the arm portion of the trigger arm 72 to deflect towards the internal space of the housing, thus driving the second fit protrusion 74 at the end portion of the trigger arm 72 to move away from the second cover 30. When the distance moved by the second fit protrusion 74 exceeds the height of the second protrusion 35 of the second cover 30, the movement of the second cover 30 is no longer constrained, and the pre-tensioned torsion spring begins to reset. As a result, the second cover 30 is driven by the driving ring 60 to rotate along the clockwise direction through the torsion arm 52 of the torsion spring. FIG. 11a and FIG. 11b illustrate that the second cover 30 has moved into the first cover 20. As can be seen from FIG. 11c, the positions of the two torsion arms of the torsion spring correspond to the positions of the torsion arms in the first state. In the fourth state, the first protrusion 25 of the first cover 20 can remain pressed against the top of the first fit protrusion 75 of the trigger arm 72, thus causing the trigger arm 72 to be in a deflected state.

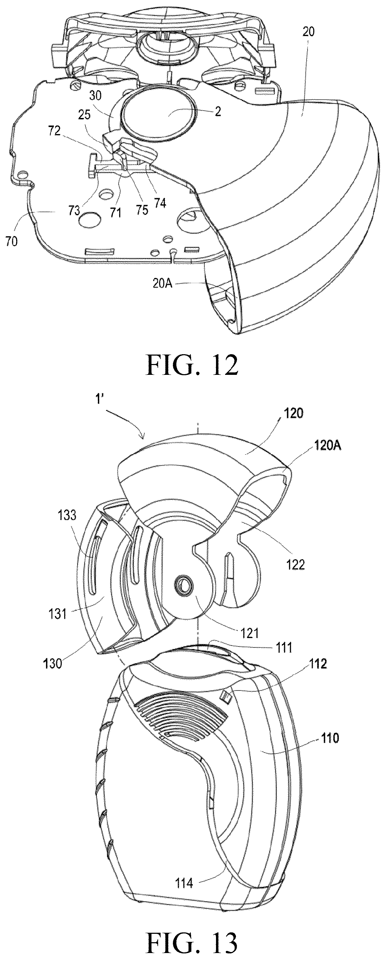

FIG 12 is the medicine dispenser 1 in an alternative fourth state. One of the differences between the alternative fourth state and the one described above is that the first protrusion 25 of the first cover 20 remains pressed against the other side portion of the first fit protrusion 75 of the trigger arm 72 so that the deflected trigger arm 72 can be reset to its original state.

In an embodiment, a top of the second protrusion 25 and/or the second fit protrusion 74 is chamfered, thus making it easier for the second protrusion 25 to slide onto the top of the first fit protrusion 75. The term "chamfer" can be understood as a rounded corner or inclined plane obtained through machining or molding. FIG. 6 illustrates an exemplary rounded corner 26 provided at the second protrusion 25.

In some instances, the side of the second fit protrusion 74 facing toward the first fit protrusion 75 is configured to be an inclined plane, and/or the side of the second protrusion 35 of the second cover 30 facing away from the second fit protrusion 74 is configured to be an inclined plane, thus making it easy for the second cover 30 to be reset to the position where the opening is closed.

FIG. 13 to FIG. 15c illustrate a medicine dispenser 1' according to an embodiment of this disclosure. Medicine dispenser 1' comprises a housing 110, a first cover 120, and a second cover 130. The structure of the medicine dispenser 1' is substantially similar to the structure of the medicine dispenser 1 described above. In the following text, only the differences of the medicine dispenser 1' will be described. For the other parts, a reference can be made to the description above more medicine dispenser 1.

The first cover 120 of the medicine dispenser 1' can be pivotally retained on the housing 110 via a pivot (not shown) arranged in the housing, so that the first cover 120 can move between a first stop position and a second stop position. In the first stop position, the first cover 120 and the second cover 130 cover the opening; and in the second stop position, the opening is released. For this purpose, at least one of side walls 121, 122 of the first cover 120 is mounted on the pivot in the housing.

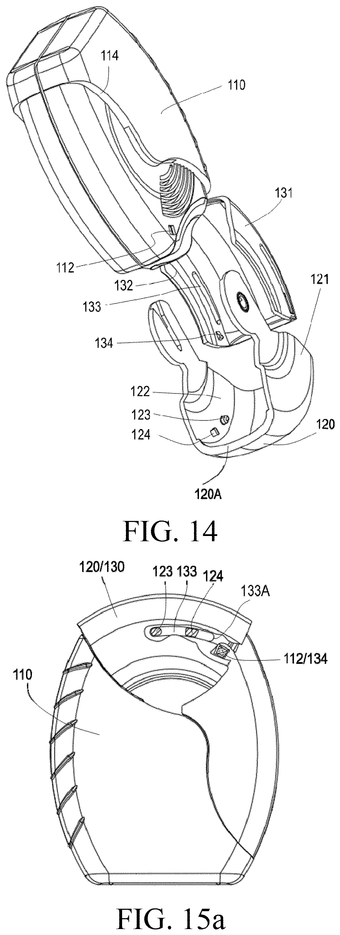

As can be seen from FIG. 13 and FIG. 14, in this embodiment, the second cover 130 is retained on the inner side of the first cover 12 rather than being connected to the pivot as described in the previous embodiments. Therefore, the first cover 120 and the second cover 130 can both be regarded as U-shaped components. The second cover 130 can also move relative to the first cover 120.

As illustrated in the drawings, the first cover 120 is provided with a first bulge 123 and a second bulge 124 on the inner sides of its side walls 121, 122, both being located along an arc, where the center of the arc falls on the pivot axis of the first cover 120. Correspondingly, the first cover 120 is provided with an arc-shaped groove 133 in the side walls 131, 132, and the radius of the arc-shaped groove corresponds to the radius of the above-described arc. The first bulge 123 is cylindrical and arranged in the arc-shaped groove 133. The second bulge 124 is positioned to enter and exit the groove 133. Referring to an opening direction of the medicine dispenser 1', e.g., a clockwise direction, the second bulge 124 is arranged downstream of the first bulge 123. The first bulge 123, the second bulge 124, and the groove 133 are configured in such a way that the circumferential distance between a front end section of the groove 133 and a front edge of the second cover is equivalent to the circumferential distance between the first bulge 123 and the second bulge 124. When the second bulge 124 moves away from the groove 133, the first bulge 123 is pressed against the front end portion of the groove, so there is no relative movement between the first cover 120 and the second cover 130, and the second cover can be driven by the first cover when the first cover continuously moves.

The end surfaces of at least two consecutive end sides of the second bulge 124 along the rotating direction can be inclined planes, thus making it easy for the second bulge 124 to leave or return to the groove.

It is to be understood that in this embodiment, the positions of the groove and the bulge arranged on the above covers and fit with each other can also be adjusted according to the need. For example, the groove can be arranged on the first cover, the bulge can be arranged on the second cover, and the bulge can also be arranged on the outer side of the second cover.

Preferably, in order to maintain the initial position of the second cover 130, the housing 110 can be provided with a limiting structure 112 for the second cover 30, and the second cover 130 can be provided with a fit limiting structure 134 corresponding to the limiting structure. For example, a recess 112 can be arranged on the housing 110, the fit limiting structure can be a protrusion 134 extend from the inner side of the second cover 130, and the protrusion can be positioned in the recess. Similarly, end surfaces of two consecutive ends of the protrusion 134 along the rotating direction can be inclined planes, thus making it easy for the protrusion 134 to leave or return to the recess.

Therefore, for medicine dispenser 1', when the first cover 120 moves to be partially misaligned with the second cover 130, the first cover 120 can drive and trigger the movement of the second cover 130. In some cases, the groove and the bulge together form a partial or whole trigger unit.

FIG. 15a to FIG. 15d respectively illustrate different states of the medicine dispenser 1'.

FIG. 15a illustrates the first state of the medicine dispenser 1', where the first cover 120 is pressed against the stop 114; the first bulge 123 and the second bulge 124 are located in the groove; and the protrusion 134 of the second cover 130 is positioned in the recess 112 of the housing. At this time, the opening 111 is covered by the above covers.

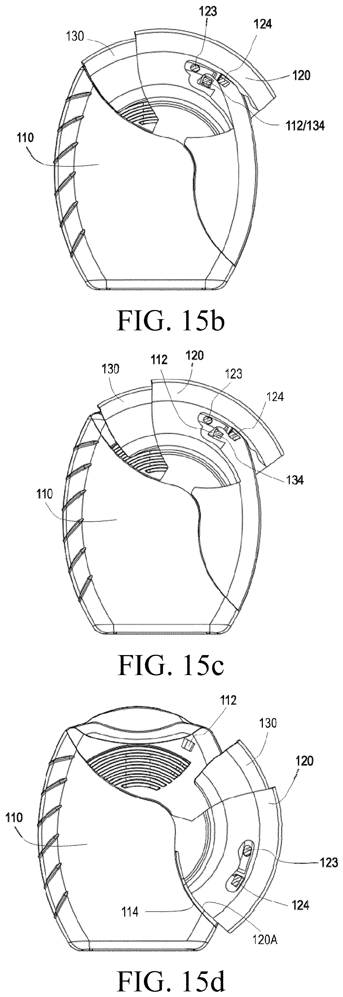

FIG. 15b illustrates the second state of the medicine dispenser 1', where the first cover 120 has rotated a first circumferential distance along the clockwise direction, causing the first bulge 123 to be pressed against the front end portion of the recess, and the second bulge 124 to be located just in front of the front edge of the second cover. In the second state, the protrusion 134 of the second cover 130 is still positioned in the recess 112 of the housing 110.

FIG 15c illustrates the third state of the medicine dispenser. At this time, the first cover 120 continuously rotates along the clockwise direction, causing the protrusion 134 of the second cover 130 to move out of the recess 112 of the housing 110 under the drive of the first cover 120.

FIG. 15d illustrates the fourth state of the medicine dispenser, where the first cover 120 rotates along the clockwise direction to approach the second stop position, and the second cover 130 fully reveals and exposes the opening 111 under the drive of the first cover 120.

In this embodiment, the groove, the bulge, the recess, and the protrusion described above can be arranged on at least one side wall of the relevant member, and are symmetrically arranged on two side walls.

In another embodiment not shown, the groove and the bulge can also be arranged for the cover in the first embodiment to establish further controlled guiding movement between the covers.

The housing 10 (and 110) can be integrally formed, or as shown in the drawings, it can include two housing halves 10A, 10B that can be assembled together.

For the convenience of manual operation, ribs 13, textured structures and the like can be arranged on the side surface of the housing opposite to the side surface through which the cover moves, so as to enhance the friction force during gripping.

The opening of the medicine dispenser can form a suction nozzle of the medicine dispenser, the first cover can be a protective cover, and the second cover can be a driving cover. The driving cover is effectively connected to the dispensing mechanism, allowing for sequential movement.

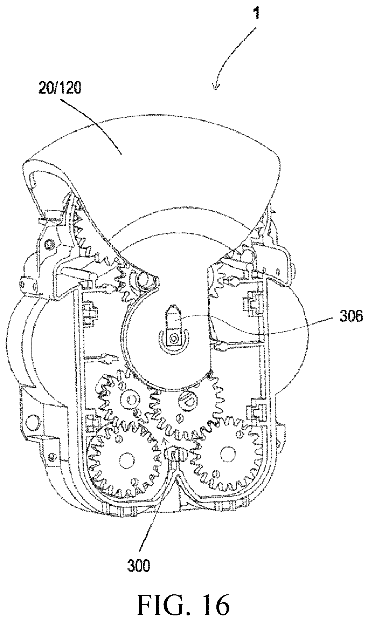

FIG. 16 is a medicine dispenser 1 comprising a housing 10 provided with a cover assembly (a first cover 20, 120 that can be seen) and a dispensing mechanism 300. As can be seen from FIG. 16, the first cover 20, 120 of the cover assembly is provided with a side wall 22, which is provided with an elongated hole for mounting to interact with a ratchet 306 of the dispensing mechanism 300 in the form of a gear set. In use, the cover assembly, i.e., the first cover and the second cover, can rotatably move around an axis defined by the rotation axis of the ratchet 306. In FIG. 16, the cover assembly is in its original position, that is, the first cover and the second cover are overlapped with each other and completely cover the opening of the housing 10.

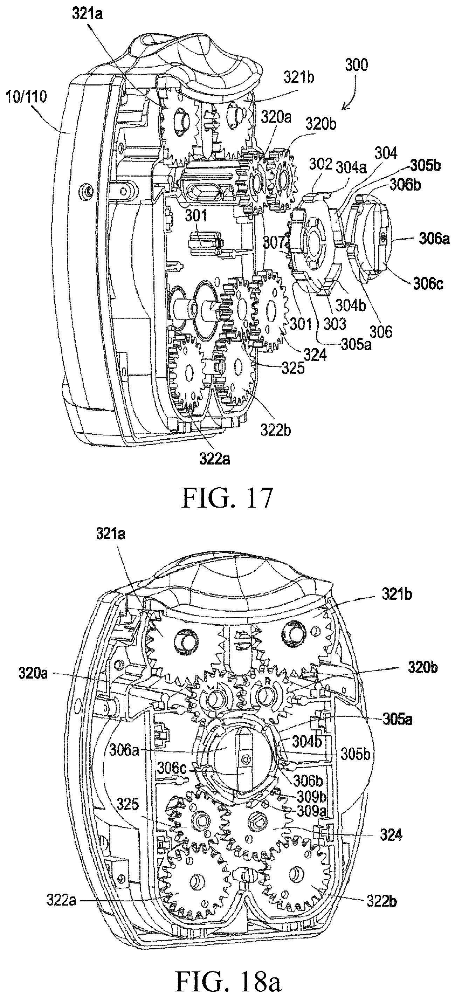

FIG. 17 is an exploded diagram of the dispensing mechanism 300 of the medicine dispenser. The dispensing mechanism 300 is positioned in the housing 10, 110. The dispensing mechanism 300 includes a ratchet gear 302 mounted on a driving spindle 301. The ratchet gear 302 has an inner surface 301 and an outer surface 303 opposite to each other (facing toward the outside of the medicine dispenser). Multiple protruding portions 304 protrude from the outer surface 303 to the outside of the housing along the circumferential direction in a manner of being spaced apart from each other, and a gear portion 307 protrudes from the inner surface 301 along the opposite direction. Each protruding portion 304 has an outer peripheral surface 305a and an inner peripheral surface 305b. The inner peripheral surface 305b is provided with a stepped profile to form a step 304b for generating a ratchet like interaction with the ratchet 306. Such interaction will be described in more detail with reference to FIG. 18a to FIG. 18c. In this embodiment, five protruding portions 304 are provided, thus forming five steps 304b in total. The steps 304b are provided in the middle of the inner peripheral surfaces 305b of the protruding portions 304, that is, are spaced apart from end portions 304a along the circumferential direction. The end portions 304a are further configured to interact with a fixed elastic pawl of the retaining plate (not shown) to prevent the ratchet 306 from returning.

The ratchet 306 includes a hub 306a in the center and multiple elastic ratchet legs 306b that are cantilevered from the outer periphery of the hub 306a in the center, are spaced apart at equal angles and are oriented along the circumferential direction, that is, one end portion of each ratchet leg 306b is connected to the hub 306a, and the other end portion is a free end portion. The hub 306a further includes a boss 306c, which is assembled into the opening of the side wall 22, 122 of the first cover as shown in FIG. 17, and is configured to form a direct driving connection between the first cover and the ratchet 306, so that the rotational movement of the first cover from its first state to the fourth state causes the rotation of the ratchet 306 in the ratchet gear 302, as will be described in more detail below. In some embodiments, the number of the elastic ratchet legs 306b corresponds to the number of the steps 304b of the ratchet gear 302.

The dispensing mechanism 300 further includes index wheels 320a, 320b, cover side winding gears 321a, 321b, bottom side winding gears 322a, 322b, and idle wheels 324, 325, which are engaged with the gear portion 307 of the ratchet gear 302. Specifically, the gear portion 307 arranged on the inner surface 301 of the ratchet gear 302 is configured to be engaged with the first index wheel 320a of the index wheels and the first idle wheel 324 of the idle wheels. The first index wheel 320a is engaged with the first cover side winding wheel gear 321a of the cover side winding wheel gears and the second index wheel 321b, and the cover side winding wheel gear is engaged with the second cover side winding wheel gear 321b. The first idle wheel 324 is engaged with the first bottom side winding gear 322b of the bottom side winding gear and the second idle gear 325, and the second idle gear 325 is engaged with the second bottom side winding gear 322a. Such arrangement provides indexing of a medicine carrier not shown and upward rolling of a base sheet and a cover sheet when the cover moves from its second position to its third position.

Referring to FIG. 18a to FIG. 18c, interactions between the ratchet gear 302 and the ratchet 306 illustrate the movement of components of the dispensing mechanism 300 when ready for use in continuous operation.

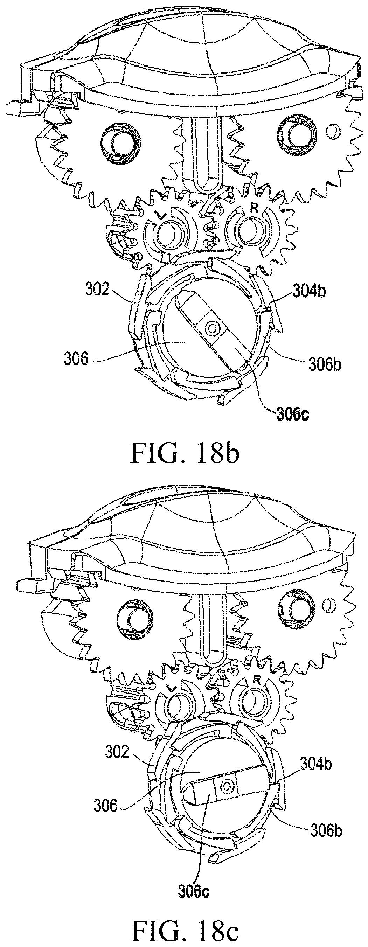

In a first position in FIG. 18a (where the cover is closed, corresponding to the first state described above), the ratchet 306 is placed in the ratchet gear 302, causing the steps 304b of the ratchet gear 302 to be spaced apart from the free end portions of the ratchet legs 306b in the circumferential direction. In the second position in FIG. 18b (where the cover is partially open, corresponding to the second state described above), the ratchet 306 rotates in the ratchet gear 302, causing the ratchet legs 306b to slide sequentially through a second section 309b of the inner peripheral surface 305b, gaps between the protruding portions 304, and a first section 309a of the inner peripheral surface 305b, thus being joined with the steps 304b. Therefore, in the second position, the ratchet gear 302 is ready to move but has not yet moved, and the dispensing mechanism 300 has not been pushed to move. In the third position in FIG. 18c (where the cover is fully open, corresponding to the fourth state described above), during the transition from the second position to the third position, the ratchet 306 and the ratchet gear 302 rotate together through the joining between the ratchet legs 306b and the steps 304b, thus pushing the dispensing mechanism 300 to move, so that the medicine carrier that interacts with the dispensing mechanism 300 opens each capsule of the carrier, thus allowing the medicine powder contained in each opened capsule to be inhaled by the user through the opening.

When closing the cover, the reverse rotation of the ratchet 306 is not transmitted to the ratchet gear 302, and thus transmitted to other gears. Therefore, each time the cover is fully opened and closed, the ratchet gear 302 only rotates in one rotation direction. When the cover returns to its original position (FIG. 1 and FIG. 16) to rotate the ratchet 306 in the ratchet gear 302 back to its original position (FIG. 18a), the elastic ratchet legs 306b can slide on the inner peripheral surface 305b, so as to be spaced apart from different steps 304b and be ready for the next opening of the cover.

According to the first embodiment, when the driving cover is in the first state, the entire opening and the protective cover are covered by the driving cover; when the driving cover is in the second state, the entire opening is covered by the protective cover, while the protective cover is partially covered by the driving cover; when the driving cover is in the third state, the entire opening is covered by the protective cover, while the protective cover is completely exposed; and when the driving cover is in the fourth state, the opening is completely exposed, while the protective cover is completely covered by the driving cover. During this period, when the driving cover moves from the first state to the second state, the movement of the protective cover and the working of the dispensing mechanism are not triggered; when the driving cover moves from the second state to the third state, any action that causes the driving cover to move from the third state to the fourth state will drive the dispensing mechanism to perform corresponding work, but the movement of the protective cover is not triggered; and when the driving cover moves from the third state to the fourth state, the movement of the protective cover is triggered.

According to the second embodiment, when the driving cover is in the first state, the entire opening and the protective cover are covered by the driving cover; when the driving cover is in the second state, the entire opening is covered by the protective cover, while the protective cover is partially covered by the driving cover; when the driving cover is in the third state, the opening is partially exposed, most of which is covered by the protective cover, and the protective cover moves along with the driving cover while being partially covered by the driving cover; and when the driving cover is in the fourth state, the opening is completely exposed, while the protective cover is partially covered by the driving cover. During this period, when the driving cover moves from the first state to the second state, the movement of the protective cover and the working of the dispensing mechanism are not triggered; when the driving cover moves from the second state to the third state, the movement of the protective cover is triggered, but the working of the dispensing mechanism is not triggered; and any action that causes the suction nozzle cover to move from the third state to the fourth state will drive the dispensing mechanism to perform corresponding work.

Although a flat medicine dispenser and its housing are shown in the drawings, they can also have any other suitable shapes, and the configurations of the covers, especially the side walls of the covers, can be correspondingly provided, as long as the above movements can be achieved.

It is to be understood that in order to simplify the drawings, only the components of the medicine dispenser related to this application are shown in the drawings, and the medicine dispenser can also have other parts.

It is to be understood that the features of the apparatus according to this disclosure or combinations of the features described above, as well as the features and combinations of the features mentioned in the drawings and/or only shown in the drawings, not only can be used according to corresponding combinations provided, but also can be used according to other combinations or used alone without departing from the scope of this disclosure.

This disclosure has been described through the above embodiments, but it is to be understood that the above embodiments are only for the purposes of giving examples and making description, and are not intended to limit this disclosure to the scope of the embodiments described. Those skilled in the art should understand that more variations and modifications can be made based on the teaching of this disclosure, all of which still fall within the scope of protection of this disclosure.

## Claims

1. A medicine dispenser (1, 1'), the medicine dispenser (1, 1') comprising:
a housing (10, 110) comprising an opening (11, 111);
a dispensing mechanism (300) positioned in the housing (10, 110) and configured to trigger medicine dispensing; and
a cover assembly configured for the opening (11, 111) and movably mounted on the housing (10, 110),
wherein the cover assembly comprises:
a first cover (20, 120), the first cover (20, 120) being pivotally mounted on the housing (10, 110); and
a second cover (30, 130), the second cover (30, 130) being arranged inside the first cover (20, 120),
wherein the second cover (30, 130) is configured to be capable of being driven by the first cover (20, 120) to pivot.

2. The medicine dispenser (1, 1') of claim 1, wherein at least before the second cover (30, 130) is driven by the first cover (20, 120), the dispensing mechanism is disengaged.

3. The medicine dispenser (1, 1') of claim 2, wherein the housing (10, 110) is provided with a stop (14, 114) for the first cover (20, 120) to provide a first stop position and a second stop position for the first cover (20, 120), and at least in the first stop position, the first cover (20, 120) completely covers the second cover (30, 130).

4. The medicine dispenser (1, 1') of claim 3, wherein the second cover (30) is pivotally mounted on the housing (10, 110), and when the first cover (20) is about to move to the second stop position, the rotational movement of the second cover (30) is triggered by a trigger unit of the medicine dispenser.

5. The medicine dispenser (1, 1') of claim 4, wherein the trigger unit comprises a torsion spring (50), two torsion arms of the torsion spring (50) are effectively connected to the first cover (20) and the second cover (30) respectively, and the torsion spring (50) is configured to respectively apply forces in opposite directions along the circumferential direction to the first cover (20) and the second cover (30).

6. The medicine dispenser (1, 1') of claim 5, wherein the trigger unit comprises a cantilevered trigger arm (72), the trigger arm (72) is elastically deformable and provided with a first fit protrusion (75) assigned to a first protrusion (25) of the first cover (20) and a second fit protrusion (74) assigned to a second protrusion (35) of the second cover (30), and the first fit protrusion (75) and the second fit protrusion (74) are spaced apart from each other.

7. The medicine dispenser (1, 1') of claim 6, wherein
in the first stop position, the second protrusion (35) of the second cover (30) is pressed against the second fit protrusion (74) of the trigger arm (72); and
in the second stop position, the first protrusion (25) of the first cover (20) is pressed against the first fit protrusion (75) of the trigger arm (72).

8. The medicine dispenser (1, 1') of claim 6, wherein the trigger unit comprises an actuator (40) non-rotatably connected to the first cover (20) and a driving ring (60) non-rotatably connected to the second cover (30), the actuator (40) is provided with a first notch (44) for a first torsion arm (51) of the torsion spring (50), and the driving ring (60) is provided with a second notch (64) for a second torsion arm (52) of the torsion spring (50).

9. The medicine dispenser (1, 1') of claim 6, wherein
the top of the second protrusion (35) and/or the second fit protrusion (74) is provided with a chamfer, and/or
the side of the second fit protrusion (74) facing toward the first fit protrusion (75) is configured to be an inclined plane, and/or
the side of the second protrusion (35) of the second cover (30) facing away from the second fit protrusion (74) is configured to be an inclined plane.

10. The medicine dispenser (1, 1') of any one of claims 6 to 9, wherein the trigger arm (72) is integral with a retaining plate (70) mounted on the housing (10, 110).

11. The medicine dispenser (1, 1') of any one of claims 1 to 9, wherein the first cover (20) is configured to be U-shaped, and the second cover (30) is configured to be hook-shaped.

12. The medicine dispenser (1, 1') of claim 3, wherein when the first cover (20, 120) moves to be partially misaligned with the second cover (30), the movement of the second cover (30, 130) is triggered by a trigger unit of the medicine dispenser.

13. The medicine dispenser (1, 1') of claim 12, wherein the second cover (30) is movably retained on the inner side of the first cover (20, 120) relative to the first cover (20, 120).

14. The medicine dispenser (1, 1') of claim 13, wherein the first cover (120) and the second cover (130) are respectively provided with an arc-shaped groove (133) and a first bulge (123) that are fit with each other, and the first bulge (123) is arranged in the arc-shaped groove (133).

15. The medicine dispenser (1, 1') of claim 14, wherein the cover with the first bulge (123) is further provided with a second bulge (124), the second bulge (124) is arranged to be capable of entering and exiting the arc-shaped groove (133), and referring to the opening direction of the medicine dispenser (1, 1'), the circumferential distance between the front end portion of the arc-shaped groove (133) and a front edge of the cover with the arc-shaped groove (133) is approximately equal to a circumferential distance between the first bulge (123) and the second bulge (124).

16. The medicine dispenser (1, 1') of claim 15, wherein the first bulge (123) is cylindrical, and the end surfaces of at least two consecutive end sides of the second bulge (124) along the opening direction are inclined planes.

17. The medicine dispenser (1, 1') of any one of claims 12 to 15, wherein the housing (110) is provided with a recess (112) for the second cover (30), and the second cover (130) is provided with a protrusion (134) corresponding to the recess (112).
